# EUROPÄISCHE PATENTSCHRIFT

(11) **EP 1 432 377 B1**
(45) Veröffentlichungstag und Bekanntmachung des Hinweises auf die Patenterteilung: **10.01.2007**
(21) Anmeldenummer: 02777270.6
(22) Anmeldetag: 01.10.2002
(51) Int. Cl.: A61H 9/00

(54) **FITNESSGERÄT**
FITNESS DEVICE
APPAREIL DE CONDITIONNEMENT PHYSIQUE

(30) Priorität: 02.10.2001 DE 10149418
(43) Veröffentlichungstag der Anmeldung: 30.06.2004
(73) Patentinhaber: Egger, Norbert, Dr., 5020 Salzburg (AT)
(72) Erfinder: Egger, Norbert, Dr., 5020 Salzburg (AT)
(74) Vertreter: Grünecker, Kinkeldey, Stockmair & Schwanhäusser Anwaltssozietät
(86) Internationale Anmeldenummer: PCT/EP2002/010980
(87) Internationale Veröffentlichungsnummer: WO 2003/030808

(56) Entgegenhaltungen:
- WO-A-01/52787
- AT-B- 400 305
- DE-A- 2 829 082
- DE-C- 843 887
- FR-A- 2 789 893
- US-A- 3 859 989
- US-A- 4 738 249
- US-A- 5 458 562

## Beschreibung

Die Erfindung betrifft ein Fitnessgerät in Form eines Bekleidungsstückes, mit einer in einem Stimulationsbereich auf die Hautoberfläche eines Trägers des Fitnessgeräts einwirkenden Hautstimulationseinrichtung mit wenigstens einer als Hohlkammer ausgestalteten Druckkammer, durch die in einem Unterdruckzustand im Stimulationsbereich ein auf die Hautoberfläche einwirkender Unterdruck erzeugbar ist und die vom Unterdruckzustand in einen Überdruckzustand, in dem im Stimulationsbereich Überdruck auf die Hautoberfläche einwirkt, überführbar ausgestaltet ist.

Bereits seit über 5000 Jahren ist es bekannt, dass eine Unterdruckeinwirkung auf die Hautoberfläche gesundheitsfördemd ist. In Urzeiten wurden Kuhhömer mit einem Loch verwendet, um Luft abzusaugen und durch Unterdruckeinwirkung im Stimulationsbereich Gewebsflüssigkeit anzusammeln. Später wurden anstelle der Kuhhömer Schröpfgläser verwendet, die mit heißer Luft gefüllt waren. Bei Abkühlung zog sich die Luft zusammen und bildete einen Unterdruck.

In der neueren Zeit wurde diese Methode weiter entwickelt und zum Teil im Firmenbereich angewendet.

So ist aus der US 726,791 ein Unterdruckanzug bekannt, der starre Abstandshalter aufweist. Nachteilig bei diesem Anzug ist jedoch die mangelnde Bewegungsfreiheit aufgrund der starren Abstandshalter, mit denen die Hülle des Anzuges beabstandet von der Hautoberfläche gehalten wird. Außerdem ist bei dem Anzug der US 726,791 eine gezielte Beeinflussung von bestimmten Körperbereichen nicht möglich.

In der US 1,440,157 ist ein gürtelähnliches Fitnessgerät gezeigt, das eine Unterdruckzone im Bauchbereich ausbildet. Bei diesem Gerät wird der Unterdruck durch die elastischen Kräfte einer sich nach vorne wölbenden Membran erzeugt.

In der US 4,230,114 ist eine Fitnesshose gezeigt, in der ein Unterdruck erzeugt wird. Um den Unterdruck einigermaßen gleichmäßig in der Fitnesshose zu verteilen, sind im Bereich der Absaugleitungen feste Abstandshalter vorgesehen.

Die Vorrichtungen der US 1,440,157 und der US 4,230,114 erlauben keine selektive Anpassung des Unterdruckbereichs an bestimmte Körperzonen.

Aus der US-A-4738249 ist ein Gerät bekannt, bei welchen Hautstimulationsvorrichtungen der Reihe nach unten Überdruck gesetzt werden können, und bei denen der Überdruck durch gemeinsame Belüftung abgebaut werden kann.

In der vom Anmelder stammenden WO 01/52787 schließlich ist ein Fitnessgerät in Form eines Bekleidungsstückes beschrieben, bei dem in einer Unterdruckkammer, die an einer Seite vom Körper eines Trägers des Bekleidungsstückes begrenzt ist, ein auf die Hautoberfläche wirkender Unterdruck erzeugt ist. Damit die Hülle des Fitnessgerätes nicht am Körper anliegt, sind Abstandshalter vorgesehen, die aufblasbar sein können. Zur Anpassung der mit Unterdruck beaufschlagten Körperbereiche an einzelne Körperabschnitte können bei dem Fitnessgerät der WO 01/52787 auch mehrere Unterdruckkammem vorgesehen sein.

Obwohl mit der Vorrichtung der WO 01/52787 bereits eine sehr gute fitnessfördemde Wirkung erreicht wird, ist dennoch eine Steigerung dieser Wirkung möglich.

Ausgehend von der WO 01/52787 liegt der Erfindung daher die Aufgabe zugrunde, die fitnesssteigemde Wirkung eines Fitnessgeräts mit den eingangs genannten Merkmalen zu steigern.

Diese Aufgabe wird für das gattungsgemäße Fitnessgerät dadurch gelöst, dass eine Mehrzahl von unabhängig voneinander vom Unterdruckzustand in den Überdruckzustand überführbaren Hautstimulationseinrichtungen und durch eine Steuereinrichtung, durch welche die Abfolge des Umschaltens einzelner Hautstimulationseinrichtungen vom Unterdruckzustand in den Überdruckzustand koordinierbar ist.

Durch diese erfindungsgemäße Lösung wird die fitnesssteigemde Wirkung des Fitnessgeräts gegenüber den herkömmlichen Vorrichtungen erhöht. Zunächst wird, wie bisher auch, im Unterdruckzustand Gewebsflüssigkeit mit Blut und Lymphflüssigkeit in das Unterhaut-Fettgewebe des Stimulationsbereichs gesaugt. Dadurch steigt im Stimulationsbereich der Stoffwechsel und die Zelltätigkeit.

Durch das Umschalten in den Überdruckzustand wird die Gewebsflüssigkeit durch den Überdruck aus dem Stimulationsbereich gepresst. Die dadurch entstehende Massagewirkung des Gerätes erhöht den durchblutungssteigernden Effekt und damit die Wirkung des Geräts gegenüber den aus dem Stand der Technik bekannten Geräten.

Die Begriffe "Überdruck" und "Unterdruck" beziehen sich dabei auf einen Druck, der höher bzw. niedriger als der Umgebungsdruck ist.

Die fitnesssteigemde Wirkung des erfindungsgemäßen Fitnessgeräts wird dadurch verbessert, dass mehrere Hautstimulationseinrichtungen vorgesehen sind, die mit der Steuereinrichtung verbunden sind und unabhängig voneinander vom Unterdruckzustand in den Überdruckzustand überführbar sind. Diese Ausgestaltung ermöglicht aufgrund der unabhängigen Ansteuerung der Hautstimulationseinrichtungen eine Anpassung des stimulierten Hautbereichs an die Körperverhältnisse des Trägers des Fitnessgeräts. So können bei dieser Ausgestaltung bestimmte Problemzonen gezielt beeinflusst werden. Die Steuereinrichtung kann einen Regler aufweisen, durch den die Abfolge der Umschaltsignale an einzelne Hautstimulationseinrichtungen einstellbar ist. Auf diese Weise kann die Reihenfolge der Umschaltvorgänge vom Unterdruckzustand in den Überdruckzustand und umgekehrt koordiniert werden. Durch diese Ausgestaltung ist es möglich, dass beispielsweise der mit Überdruck beaufschlagte Stimulationsbereich durch gezieltes Ansteuern der Hautstimulationseinrichtungen und durch eine gezielte Abfolge der Umschaltsignale, an die einzelnen Hautstimulationseinrichtungen entlang bestimmter Wege über die Hautoberfläche gesteuert werden kann. So lässt sich eine gezielte Massagewirkung erreichen, die an die anatomischen Verhältnisse des menschlichen Körpers angepasst ist. Beispielsweise kann sich der Überdruckbereich in Richtung der Blutbahnen zum Herzen hin oder vom Herzen weg steuern, oder der Stimulationsbereich nur über bestimmte Muskeln bewegt werden.

In einer Weiterbildung können auch einzelne der Mehrzahl von Halbstimulationseinrichtungen so gekoppelt werden, dass sie stets gleichzeitig auf das Umschaltsignal reagieren.

Die Hautstimulationseinrichtungen weisen eine Druckkammer auf, die im Überdruckzustand mit einem Kammerdruck beaufschlagt ist, der größer als der Umgebungsdruck ist und/oder die im Unterdruckzustand mit einem Kammerdruck beaufschlagt ist, der niedriger als der Umgebungsdruck ist. Derartige Druckkammern lassen sich auf einfache Weise durch Vakuumpumpen und Ventilsteuerung ansteuern.

Die Druckkammern sind als Hohlkammern ausgestaltet, die in Abhängigkeit vom Umschaltsignal mit der Saugseite und/oder der Druckseite und/oder einem mit der Umgebung verbundenen Auslass einer Fluidpumpe verbindbar sind. Je nachdem mit welchem Ausgang der Pumpe die Druckkammer verbunden ist, wird sie mit einem Unterdruck oder einem Überdruck beaufschlagt.

Im Überdruckzustand kann sich die Hautstimulationseinrichtung insbesondere an der Hautoberfläche abstützen. Bei dieser Ausgestaltung dient die Hautstimulationseinrichtung im Überdruckzustand als Abstandshalter. So kann in einer weiteren vorteilhaften Ausgestaltung im Unterdruckzustand die Hautstimulationseinrichtung von der Hautoberfläche beabstandet sein.

Die Hohlkammer kann in einer weiteren Ausgestaltung zumindest abschnittsweise eine flexible Hülle aufweisen, so dass sie sich im Überdruckzustand ausdehnt und im Unterdruckzustand zusammenzieht.

In einer anderen vorteilhaften Ausgestaltung kann die Hautstimulationseinrichtung auch ein elastisches Element aufweisen, durch das im Überdruckzustand eine Druckkraft auf den Stimulationsbereich ausgeübt wird. Das elastische Element kann beispielsweise in Form von federnden Spangen, die die Druckkammer aufspannen oder in Form von Federn, die sich auf der Hautoberfläche des Träger des Fitnessgeräts abstützen, ausgestaltet sein. Wird bei dieser Ausgestaltung die Hautstimulationseinrichtung mit einem Unterdruck beaufschlagt, so wirkt der Unterdruck gegen die Druckkraft der elastischen Elemente und drückt diese zusammen. Durch die elastischen Elemente kann auf die Erzeugung eines Überdrucks im Überdruckzustand verzichtet werden, was den Aufbau des Fitnessgeräts erheblich vereinfacht.

Das Fitnessgerät kann ferner mit einer Magnetfeldeinrichtung ausgestattet sein, durch die ein auf den Körper des Trägers des Fitnessgeräts einwirkendes Magnetfeld erzeugbar ist. Durch das Magnetfeld lassen sich positive Wirkungen im Körper des Trägers des Fitnessgerätes erreichen.

Außerdem kann gemäß einer weiteren vorteilhaften Ausgestaltung auch eine Bestrahlungseinrichtung vorgesehen sein, durch die der Körper des Trägers zumindest abschnittsweise mit Licht im sichtbaren Bereich oder im Infrarot- oder Ultraviolettbereich bestrahlt wird. Durch Lichtleiter, beispielsweise Glasfaserkabel, kann gemäß einer vorteilhaften Weiterbildung das Licht von einer Lichtquelle zu bestimmten Bereichen des Trägers geleitet werden. Dies ermöglicht größere Freiheiten in der Ausgestaltung des Fitnessgeräts, da die Lichtquelle an beliebigen Stellen angebracht werden kann. Durch die Bestrahlung der Hautoberfläche mit Infrarotstrahlen lässt sich eine lokale Erwärmung der Haut erreichen. Durch bestimmte Wellenlängen von Lichtstrahlen kann der Hormonhaushalt in der Hautoberfläche beeinflusst werden.

Um innerhalb des Fitnessgeräts ein für den jeweiligen Einsatzzweck förderliches Klima zu erhalten, kann gemäß einer vorteilhaften Weiterbildung eine Klimatisierungseinrichtung vorgesehen sein, durch die die Feuchte und/oder die Temperatur in dem an die Hautoberfläche angrenzenden Bereich des Fitnessgeräts zumindest abschnittsweise regelbar ist. Diese Regelung kann beispielsweise durch die Steuerung der Feuchte des in das Fitnessgerät eingeleiteten Fluids oder dessen Temperatur geschehen. Als Fluid eignet sich in einer kostengünstigen Variante des Fitnessgeräts insbesondere Umgebungsluft. Es können aber auch andere Gase, beispielsweise Edelgase oder Gasgemische verwendet werden, die bei Hautkontakt fitnessfördemde Wirkung haben.

Um dem Träger des Fitnessgeräts größere Bewegungsfreiheit zu geben und es ihm gleichzeitig zu ermöglichen, dass er sich während des Betriebs des Fitnessgeräts sportlich, beispielsweise in einem Fitnessstudio, betätigt, ist das als Bekleidungsstück ausgestattete Fitnessgerät flexibel und bewegungsfolgend ausgestaltet, so dass es den Bewegungen seines Trägers ohne größere Behinderungen folgen kann. Hierzu kann das Fitnessgerät insbesondere eine flexible, im Stimulationsbereich vorzugsweise fluidundurchlässige Hülle aufweisen. Das Fitnessgerät dient insbesondere bei gleichzeitiger sportlicher Betätigung als Schutzanzug. Durch eine entsprechende Innenbeschichtung, die ein angenehmes Tragegefühl vermittelt, beispielsweise ein Vliesmaterial, wird der Tragekomfort nochmals erhöht.

Zur Steigerung der fitnessfördernden und schlankmachenden Wirkung des Fitnessgeräts ist erfindungsgemäß vorgesehen, dass die Hautstimulationseinrichtung abwechselnd einen Unterdruck und einen Überdruck auf den Stimulationsbereich ausübt.

Das Fitnessgerät kann in Form einer Hose, in Form eines Hemdes, eines Ganzkörperanzuges oder in Form von Arm- oder Bein-Bandagen oder Leibbinden ausgestaltet sein.

Im Folgenden wird das erfindungsgemäße Fitnessgerät mit Bezug auf die beigefügten Figuren anhand von Ausführungsbeispielen näher erläutert. Dabei werden für gleiche Bauelemente die gleichen Bezugszeichen verwendet.

Es zeigen:
- Figur 1: Ein erstes Ausführungsbeispiel eines erfindungsgemäßen Fitnessgeräts;
- Figur 2: ein zweites Ausführungsbeispiel eines erfindungsgemäßen Fitnessgeräts;
- Figur 3 A: ein drittes Ausführungsbeispiel eines erfindungsgemäßen Fitnessgeräts;
- Figur 3 B: das Ausführungsbeispiel der Figur 3 A im Querschnitt;
- Figur 4: ein viertes Ausführungsbeispiel eines erfindungsgemäßen Fitnessgeräts;
- Figur 5 A: ein fünftes Ausführungsbeispiel eines erfindungsgemäßen Fitnessgeräts;
- Figur 5 B: ein Querschnitt durch das Ausführungsbeispiel der Figur 5 A;
- Figur 6 A: ein sechstes Ausführungsbeispiel des erfindungsgemäßen Fitnessgeräts in einem ersten Betriebszustand;
- Figur 6 B: das Ausführungsbeispiel der Figur 6 A in einem zweiten Betriebszustand;
- Figur 6 C: einen Querschnitt durch die Figur 6 A;
- Figur 6 D: einen Querschnitt durch die Figur 6 B;
- Figur 7 A: das Ausführungsbeispiel der Figur 6 A in einem weiteren Betriebszustand;
- Figur 7 B: das Ausführungsbeispiel der Figur 7 A in einem weiteren Betriebszustand;
- Figur 7 C: das Ausführungsbeispiel der Figur 7 A in einem weiteren Betriebszustand;
- Figur 7 D: einen Querschnitt durch den Betriebszustand der Figur 7 A;
- Figur 7 E: einen Querschnitt durch den Betriebszustand der Figur 7 B;
- Figur 7 F: einen Querschnitt durch den Betriebszustand der Figur 7 C;
- Figur 8 A: ein siebtes Ausführungsbeispiel des erfindungsgemäßen Fitnessgeräts in einer ersten Betriebsart;
- Figur 8 B: das Ausführungsbeispiel der Figur 8 A in einer zweiten Betriebsart;
- Figur 9: ein achtes Ausführungsbeispiel des erfindungsgemäßen Fitnessgeräts;
- Figur 10: ein neuntes Ausführungsbeispiel des erfindungsgemäßen Fitnessgeräts;

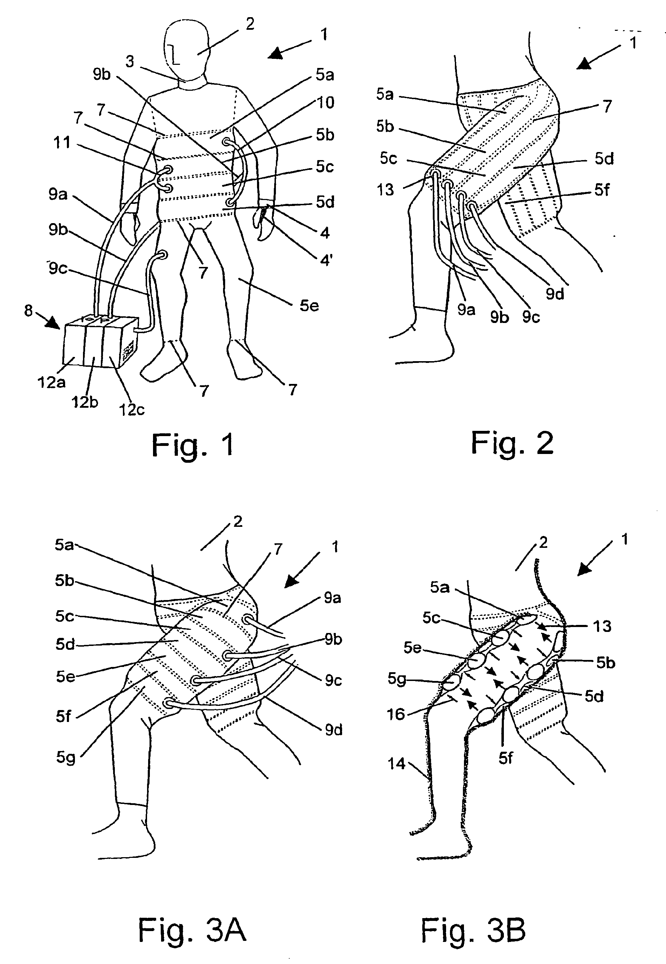

Zunächst wird der Aufbau eines ersten Ausführungsbeispiels des erfindungsgemäßen Fitnessgeräts anhand der Figur 1 beschrieben.

Figur 1 zeigt ein Fitnessgerät 1 in Form eines Bekleidungsstückes, das von einem nur schematisch dargestellten Träger 2 getragen wird. Das Fitnessgerät 1 weist die Form eines Ganzkörperanzugs oder Overalls auf, der mit einem Kragen 3 und Bünden 4 an den Armen versehen ist. Die Bünde 4 dichten den Innenraum des Fitnessanzuges 1 ab. Daumenschlaufen 4' verhindern ein Verrutschen des Anzuges.

Der Fitnessanzug 1 ist mit sich um den gesamten Körperumfang des Trägers 2 erstreckenden, im wesentlichen schlauchförmigen Hautstimulationseinrichtungen 5 a - d versehen. Wird im folgenden lediglich generisch auf die Hautstimulationseinrichtungen Bezug genommen, so werden die Buchstaben weggelassen. Im Bereich der Beine ist eine weitere Hautstimulationseinrichtung 5 e vorgesehen.

Der Bereich jeder Hautstimulationseinrichtung 5 definiert einen Stimulationsbereich auf der Hautoberfläche des Trägers 2, in dem die Hautstimulationseinrichtung auf den Körper des Trägers 2 mittels Unterdruck oder Überdruck einwirkt. Die Hautstimulationsbereiche 5 a - d sind voneinander durch Trennbereiche 7 getrennt. Durch die Trennbereiche 7 sind die Stimulationsbereiche so voneinander isoliert, dass eine Hautstimulationseinrichtung 5 jeweils nur den ihr zugeordneten Stimulationsbereich beeinflusst.

Beim Ausführungsbeispiel der Figur 1 sind im Rumpfbereich insgesamt vier Hautstimulationseinrichtungen 5 a - d vorgesehen. Die an den Beinen angeordnete Hautstimulationseinrichtung 5 e wirkt auf beide Beine gleichzeitig. Die Anzahl der Hautstimulationseinrichtungen 5 kann je nach Bedarf und Größe des Anzuges variiert werden.

Die Hautstimulationseinrichtungen der Figur 1 werden durch einen Fluiddruck betätigt, der von einer Pumpenvorrichtung 8 erzeugt wird. Als Fluid wird beim Ausführungsbeispiel der Figur 1 ein Gas, beispielsweise ein inertes Gas wie Helium oder Kohlendioxid oder eine Mischung von Gasen, beispielsweise Umgebungsluft, verwendet. Für bestimmte Anwendungen können jedoch auch Flüssigkeiten als Fluide verwendet werden.

Der von der Pumpenvorrichtung 8 erzeugte Fluiddruck, d.h. ein Druck, der gegenüber dem in der Außenumgebung des Fitnessgeräts herrschenden Umgebungsdruck niedriger oder höher ist, wird über Druckzuleitungen 9 a, 9 b, 9 c an die Hautstimulationseinrichtungen weitergeleitet. Hierzu sind die Druckzuleitungen 9 a, 9 b, 9 c mit der Saugseite oder der Druckseite der Pumpenvorrichtung 8 verbindbar.

Wie in der Figur 1 zu erkennen ist, sind beim Ausführungsbeispiel der Figur 1 die oberste und die unterste Hautstimulationseinrichtung 5 a und 5 d im Rumpfbereich durch eine Verbindungsleitung 10 gekoppelt. Die Verbindungsleitung 10 ist mit der Druckzuleitung 9 b verbunden, so dass die oberste und die unterste Hautstimulationseinrichtung 5 im Rumpfbereich stets den gleichen Fluiddruck aufweisen.

Ebenso sind die beiden mittleren Hautstimulationseinrichtungen 5 b und 5 c im Rumpfbereich über eine Verbindungsleitung 11 miteinander verbunden. Die Verbindungsleitung 11 ist mit der Druckzuleitung 9 a verbunden, so dass in den beiden Hautstimulationseinrichtungen 5 b und 5 c stets derselbe Fluiddruck herrscht. Die Hautstimulationseinrichtung 5 e an den Beinen wird, wie in der Figur 1 zu sehen ist, separat über eine Druckzuleitung 9 c mit Fluiddruck beaufschlagt.

In der Pumpvorrichtung 8 ist eine Steuereinrichtung nicht dargestellt, die ein Umschaltsignal ausgibt, auf das über Schaltventile 10 a - c hin die Druckzuleitungen 9 a - c entweder mit Überdruck oder mit Unterdruck beaufschlagt werden. Somit kann jede Hautstimulationseinrichtung 5 in dem ihr zugeordneten Stimulationsbereich entweder einen Unterdruck auf die Hautoberfläche oder einen Überdruck auf die Hautoberfläche aufbringen.

Wirkt ein Unterdruck auf die Hautoberfläche, so wird Blut und Gewebsflüssigkeit im Stimulationsbereich angesammelt. Im Überdruckzustand werden das Blut und die Gewebsflüssigkeit aus dem Stimulationsbereich weggedrückt.

Das Fitnessgerät in Anzugform der Figur 1 wird folgendermaßen betrieben: Zunächst wird die Luft aus dem Anzug über die Druckleitung 9 c abgesaugt, so dass im Fitnessgerät in Unterdruck zwischen 50 und 600 Millibar herrscht. Die luftdichte Außenhaut des Anzuges presst sich mit dem entsprechenden Druck an die Haut der Person 2, die sich im Anzug 1 befindet. Die Druckleitung 9 a wird mit der Saugseite der Pumpvorrichtung 8 verbunden, während die Druckleitung 9 b mit der Druckseite der Pumpe verbunden wird. Auf diese Weise blasen sich die Hautstimulationseinrichtungen 5 a und 5 d auf, so dass die zwischen diesen Hautstimulationseinrichtungen liegenden, mit Unterdruck beaufschlagten Hautstimulationseinrichtungen 5 b und 5 c von der Haut der Person abgehoben werden. Unter den aufgeblasenen Luftkammern wirkt ein Überdruck, während unter den abgesaugten Luftkammern Unterdruck auf die Haut wirkt.

Zur Erhöhung der Wirkung können beim Ausführungsbeispiel der Figur 1 sowie auch in den folgenden Ausführungsbeispielen Vorrichtungen zur Erzeugung eines Magnetfeldes, Lichtquellen mit Glasfaserleitungen und Klimatisierungseinrichtungen zur Klimatisierung der von der Pumpenvorrichtung 8 geförderten Luft vorgesehen sein.

Figur 2 zeigt ein zweites Ausführungsbeispiel eines erfindungsgemäßen Fitnessgeräts, das in Hosenform ausgestaltet ist und so flexibel ist, dass es eine normale sportliche Betätigung des Trägers ermöglicht. Die Stimulationsbereiche erstrecken sich bei diesem Ausführungsbeispiel über den Oberschenkel-, Unterbauch- und Pobereich, so dass sich das Ausführungsbeispiel der Figur 2 besonders zum Fettabbau im Oberschenkel-, Po- und Unterbauchbereich eignet. Die den Stimulationsbereichen zugeordneten Hautstimulationseinrichtungen 5 erstrecken sich in Richtung der Beinlänge von kurz oberhalb der Knie bis zur Taille. Jede Hautstimulationseinrichtung ist durch einen Trennbereich 7 von der benachbarten Hautstimulationseinrichtung getrennt und mit einem Anschluss 13 versehen, an den eine Druckzuleitung 9 angeschlossen ist. Jede Druckzuleitung 9 ist separat und unabhängig von der Druckzuleitung 9 einer anderen Hautstimulationseinrichtung 5 mit Fluiddruck- Unterdruck oder Überdruck- beaufschlagbar. Der Einfachheit halber sind lediglich vier Druckleitungen 9 a - d in Figur 2 gezeigt, die den Hautstimulationseinrichtungen 5 a - d zugeordnet sind; die restlichen Druckzuleitungen sind weggelassen.

Beim Ausführungsbeispiel der Figur 2 ist ferner eine Klimatisierungseinrichtung (nicht gezeigt) vorgesehen, durch die Temperatur und/oder Feuchte in der Fitnesshose 1 auf einstellbare Werte regelbar sind. Dies geschieht beispielsweise durch Klimatisierung der durch die Druckzuleitungen zugeführte Druckluft.

Figur 3 A zeigt ein drittes Ausführungsbeispiel, das ebenfalls als eine Hose ausgestaltet ist. Figur 3 B zeigt das dritte Ausführungsbeispiel im Querschnitt. Im Unterschied zum Ausführungsbeispiel der Figur 2 erstrecken sich beim Ausführungsbeispiel der Figur 3 die Stimulationsbereiche mit den dazugehörigen Hautstimulationseinrichtungen in Umfangsrichtung um den die Oberschenkel und die Taille. Der Einfachheit halber ist beim Ausführungsbeispiel der Figur 3 A nur jede zweite Hautstimulationseinrichtung mit einer Druckzuleitung dargestellt.

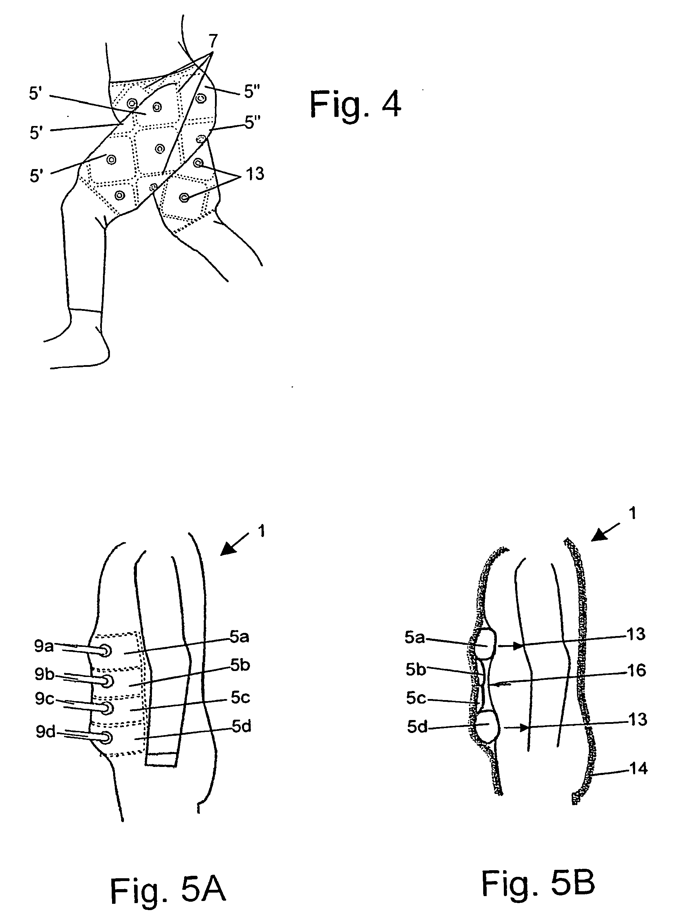

Im Querschnitt der Figur 3 B ist zu erkennen, dass die hohlkammerartigen Hautstimulationseinrichtungen 5 a, 5 c, 5 e und 5 g durch Beaufschlagung mit Überdruck aufgeblasen sind. Dadurch stützen sich die Hautstimulationseinrichtungen 5 a, 5 c, 5 e und 5 g auf der Haut ab und üben dort einen Druck aus. Somit werden mit Überdruck beaufschlagte Stimulationsbereiche 13 gebildet, die durch die körpereinwärts gerichteten Pfeile in Figur 3 B schematisch dargestellt sind. In den dazwischenliegenden hohlkammerartigen Hautstimulationseinrichtungen 5 b, 5 d und 5 e herrscht Unterdruck, so dass diese kollabieren. Da die Hülle der Hautstimulationseinrichtungen flexibel ist, sind bei den mit Unterdruck beaufschlagten Hautstimulationseinrichtungen 5 b, 5 d und 5 e die Hüllen zusammengezogen. Da die mit Unterdruck beaufschlagten Hautstimulationseinrichtungen 5 b, 5 d, 5 e an der Hülle 14 des Fitnessgeräts 1 angebracht sind und durch die aufgeblasenen Hautstimulationseinrichtungen vom Körper des Trägers 2 weggedrückt werden, bildet sich jeweils zwischen den mit Unterdruck beaufschlagten Hautstimulationseinrichtungen 5 b, 5 d und 5 e ein Hohlraum 15, der eine Sogwirkung auf die darunter liegende Hautoberfläche ausübt. Durch diese Sogwirkung werden Unterdruck-Stimulationsbereiche 16 gebildet, die in Figur 3 B durch aus dem Körper des Trägers 2 gerichtete Pfeile dargestellt sind. In Abhängigkeit von der geometrischen Ausgestaltung der Hautstimulationseinrichtung 5 kann der Stimulationsbereich an einzelne Muskelformen angepasst werden. Außerdem können durch die Hautstimulationseinrichtungen beliebige Muster zusammengesetzt werden, die ein beliebiges Zusammenwirken und eine beliebige Anpassung der Stimulationsbereiche an die Körpergeometrie erlauben. Dies ist exemplarisch in der Figur 4 dargestellt.

Beim Ausführungsbeispiel der Figur 4 sind die Stimulationsbereiche in etwa rechteckig. Sie können aber auch polygonal, rund oder abgerundet sein. Jede Hautstimulationseinrichtung 5 ist mit einem Schlauchanschluss 13 versehen und kann unabhängig angesteuert werden. Werden mehrere Hautstimulationseinrichtungen parallel mit dem gleichen Druck beaufschlagt, so wirken sie zusammen und bilden zusammenhängend einen Stimulationsbereich. Beispielsweise können beim Ausführungsbeispiel der Figur 4 die Hautstimulationseinrichtungen 5' so zusammen betrieben werden, dass sich der Stimulationsbereich nur über den vierköpfigen Oberschenkelmuskel erstreckt. Unabhängig davon können die Hautstimulationseinrichtungen 5" zusammen angesteuert werden, so dass ein weiterer Stimulationsbereich sich nur über den Gesäßmuskel erstreckt.

Figur 5 A zeigt ein fünftes Ausführungsbeispiel des erfindungsgemäßen Fitnessgeräts, Figur 5 B einen Querschnitt durch dieses Ausführungsbeispiel. Im Ausführungsbeispiel der Figur 5 A sind vier Hautstimulationseinrichtungen 5 a - d im Bauchbereich des Fitnessanzuges 1 gezeigt.

Im Querschnitt der Figur 5 B ist zu erkennen, dass die Hautstimulationseinrichtungen von Hohlkammern gebildet sind, deren Hülle 16 flexibel ist. Die flexible Hülle 16 des Fitnessanzugs ist in dieser Darstellung ebenfalls zu erkennen. Zur Schweißabsorption ist die flexible Hülle 16 innen mit einem weichen, komfortablen Vliesmaterial beschichtet.

In der Darstellung der Figuren 5 A und 5 B ist die oberste Hautstimulationseinrichtung 5 a und die unterste Hautstimulationseinrichtung 5 d mit Überdruck beaufschlagt, so dass sie sich im aufgeblasenen Überdruckzustand befinden. Die beiden mittleren Hautstimulationseinrichtungen 5 b und 5 c sind mit Unterdruck beaufschlagt, so dass sie im Unterdruckzustand zusammengezogen sind. Im Überdruckzustand stützen sich die Hautstimulationseinrichtungen 5 a und d in den Stimulationsbereichen 13 an der Hautoberfläche 19 des Trägers 2 ab und üben einen Überdruck auf das darunter liegende Gewebe aus.

Im Stimulationsbereich 16, der den beiden sich im Unterdruckzustand befindlichen Hautstimulationseinrichtungen 5 b und c zugeordnet ist, herrscht ein Unterdruck.

Die beiden aufgeblasenen Hautstimulationseinrichtungen 5 a und 5 d dienen als Abstandshalter, so dass die beiden kollabierten Hautstimulationseinrichtungen 5 von der Hautoberfläche 19 des Trägers 2 weggedrückt werden. Dadurch bildet sich zwischen den Hautstimulationseinrichtungen 5 im Unterdruckzustand und der Hautoberfläche 19 eine mit Unterdruck beaufschlagte Kammer 15.

Werden nun die beiden mittleren Hautstimulationseinrichtungen 5 b, c mit einem Überdruck beaufschlagt und die beiden am Rande gelegenen Hautstimulationseinrichtungen 5 a, d mit einem Unterdruck beaufschlagt, so kollabieren die beiden am Rande gelegenen Hautstimulationseinrichtungen 5 a, d und bilden darunter jeweils eine Kammer 15 mit einem Unterdruck-Stimulationsbereich 16, während sich die beiden mittleren Stimulationseinrichtungen an die Haut anpressen und einen Überdruck-Stimulationsbereich 13 bilden.

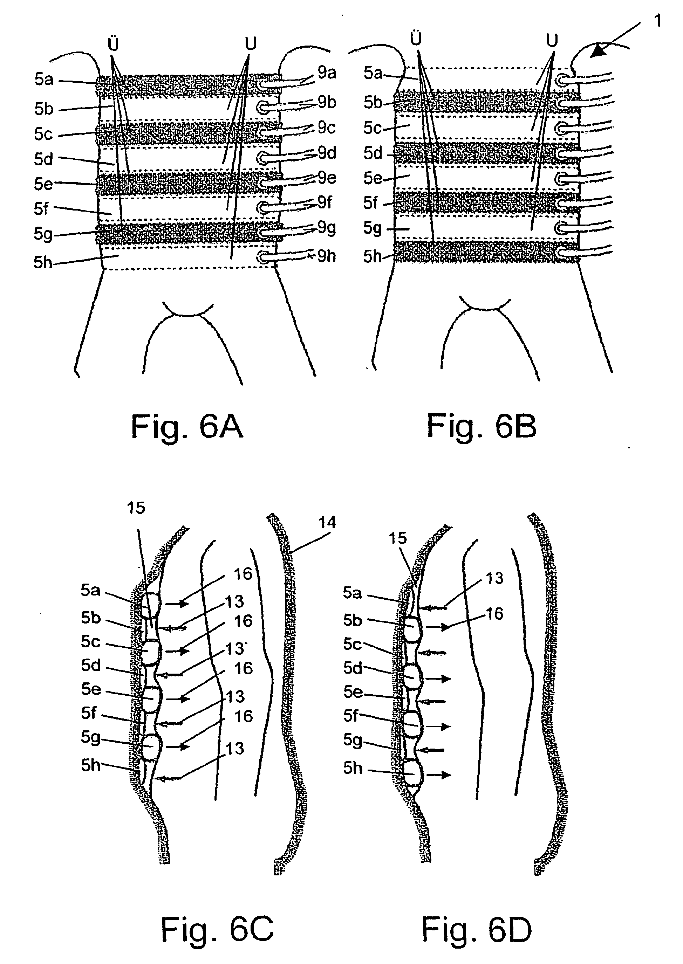

In den Figuren 6 A bis 6 D ist eine erste Betriebsart eines weiteren Ausführungsbeispiels eines erfindungsgemäßen Fitnessgeräts 1 in Bekleidungsform gezeigt. Die mit Unterdruck beaufschlagten Hautstimulationseinrichtungen sind in den Figuren 6 A bis 6 D mit U, die mit Überdruck beaufschlagten mit Ü bezeichnet. Diesen entsprechen jeweils Unterdruck- und Überdruck-Stimulationsbereiche 13 und 16. In den Figuren 6 A und 6 B ist jeweils die mit einem Überdruck beaufschlagte Hautstimulationseinrichtung dunkel dargestellt.

Das Fitnessgerät der Figuren 6 A bis 6 D ist in Form einer Leibbinde ausgestaltet, die sich vom Hüftbereich bis unter die Achseln erstreckt. Das Fitnessgerät weist insgesamt acht konzentrische, ringförmige Hautstimulationseinrichtungen 5 a - h im Rumpfbereich, denen Druckzuleitungen 9 a - h zugeordnet sind, auf. Über die Druckzuleitungen 9 a - b werden die Hautstimulationseinrichtungen mit einem Fluid- unter- oder einem Fluidüberdruck beaufschlagt. Jede zweite Hautstimulationseinrichtung ist dabei in Parallelschaltung mit der gleichen Druckquelle verbunden.

In der in Figur 6 A gezeigten Ausgangsstellung ist beginnend mit der ersten Hautstimulationseinrichtung 5 a jede zweite Hautstimulationseinrichtung 5 a, 5 c, 5 e, 5 g mit einem Überdruck beaufschlagt, während beginnend mit der zweiten Hautstimulationseinrichtung 5 b jede zweite Hautstimulationseinrichtung 5 b, 5 d, 5 f, 5 h mit einem Unterdruck beaufschlagt wird. In regelmäßigen Zeitintervallen, beispielsweise alle 5 - 40 Sekunden, vorzugsweise alle 10-20 Sekunden, wird von dem in Figur 6 A dargestellten ersten Betriebszustand in den in Figur 6 B dargestellten zweiten Betriebszustand umgeschaltet. Im Betriebszustand der Figur 6 B ist beginnend mit der obersten Hautstimulationseinrichtung 5 a jede zweite Hautsfimulationseinrichtung 5 a, 5 c, 5 e, 5 g nunmehr mit Unterdruck beaufschlagt, während beginnend mit der zweiten Hautstimulationseinrichtung 5 b von oben jede zweite Hautstimulationseinrichtung 5 b, 5 d, 5 f, 5 h nunmehr mit Überdruck beaufschlagt ist. Der Überdruck in den aufgeblasenen Kammern beträgt dabei 10-50 oder 200 - 800 Millibar, die Druckdifferenz zwischen Über- und Unterdruck beträgt bevorzugt 20-10 Millibar.

Wie in den Figuren 6 C und 6 D zu erkennen ist, die jeweils Querschnitte der Betriebszustände der Figur 6 A und der Figur 6 B darstellen, wird durch das Umschalten jeder Hautstimulationseinrichtung 5 vom Unterdruckzustand in den Überdruckzustand und umgekehrt ein entsprechender Wechsel in den Stimulationsbereichen 13, 16 hervorgerufen. Dies führt zu einer Massage des Unterhautfettgewebes.

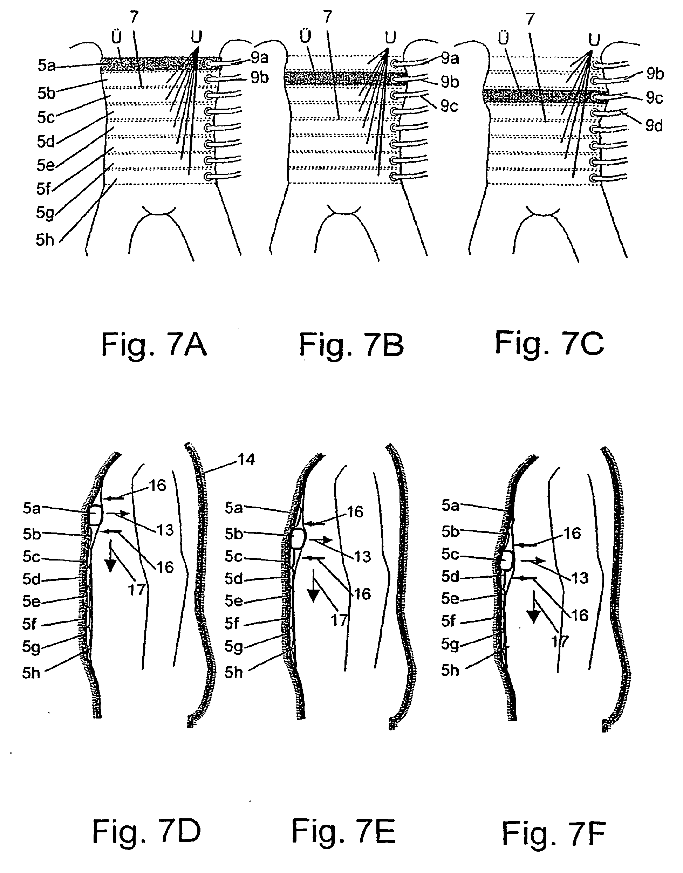

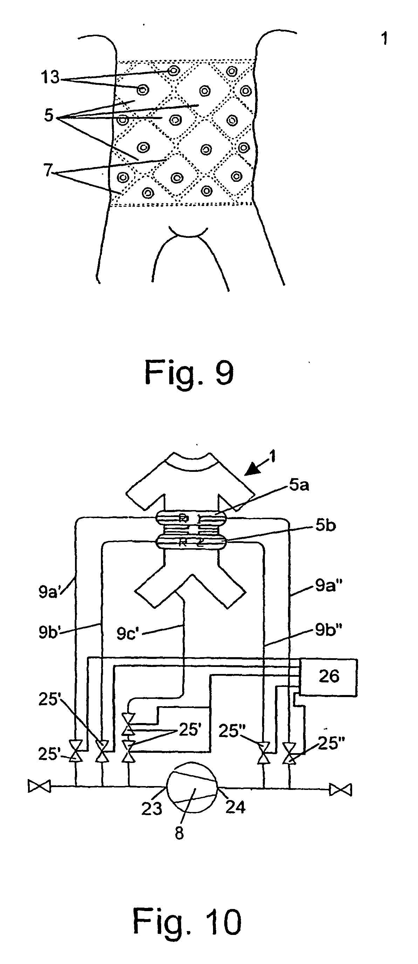

Ein weiteres Beispiel zur Steuerung der Stimulationsbereich des erfindungsgemäßen Fitnessgeräts in den Figur 7 A bis 7 F gezeigt. Dabei wird dasselbe Fitnessgerät verwendet, wie es in den Figuren 6 A bis 6 D dargestellt ist. Die Figuren 7 A bis 7 C zeigen eine Vorderansicht, die Figuren 7 D bis 7 F jeweils einen Querschnitt durch die darüber liegende Vorderansicht.

Bei den Figuren 7 A bis 7 F ist jede der Hautstimulationseinrichtungen 5 a - h unabhängig von den übrigen Hautstimulationseinrichtungen getrennt steuerbar. Dadurch ist es möglich, einen Stimulationsbereich zu erzeugen, der sich durch eine gezielte und koordinierte Ansteuerung der Hautstimulationseinrichtungen über die Körperoberfläche bewegt.

So wird in Figur 7 A zunächst die oberste Hautstimulationseinrichtung 5 a mit Überdruck beaufschlagt, während die übrigen Hautstimutationseinrichtungen 5 b - h mit Unterdruck beaufschlagt sind. Dann wird allmählich der Druck in der obersten Hautstimulationseinrichtung 5 a verringert und Druck in der benachbarten, darunter liegenden Hautstimulationseinrichtung 5 b aufgebaut, bis der in Figur 7 B gezeigte Betriebszustand erreicht ist.

Im Betriebszustand der Figur 7 B ist die oberste Hautstimulationseinrichtung 5 a mit Unterdruck und die darunter liegende Hautstimulationseinrichtung mit Überdruck beaufschlagt; die restlichen Hautstimulationseinrichtungen 5 c - h sind gegenüber dem Zustand der Figur 7 A unverändert geblieben.

Nun wird der obige Vorgang mit der jeweils darunter liegenden Hautstimulationseinrichtung so wiederholt, dass sich der mit Überdruck beaufschlagte Stimulationsbereich entlang des Fitnessgerätes 1 von oben nach unten bewegt, wie dies durch die eingeschwärzte Hautstimulationseinrichtung in den Figuren 7 A. bis 7 C dargestellt ist: Als nächstes wird in der zweiten Hautstimulationseinrichtung 5 b wird allmählich der Druck verringert und in der darunter liegenden benachbarten Hautstimulationseinrichtung 5 c Druck aufgebaut, bis der in Figur 7 C dargestellte Zustand erreicht und in der dritten Hautstimulationseinrichtung 5 c ein Überdruck vorhanden ist, während die übrigen Hautstimulationseinrichtungen 5 a - b, d - h mit Unterdruck beaufschlagt sind.

In einer Weiterbildung dieses Verfahrens können auch mehrere Stimulationsbereiche gleichzeitig den Körper entlang laufen, beispielsweise indem in Ausgangsstellung der Figur 7 A zusätzlich die fünfte Hautstimulationseinrichtung 5 e von oben ebenfalls mit Überdruck beaufschlagt ist und sich dieser Stimulationsbereich zusätzlich zu und synchron mit dem Stimulationsbereich der obersten Hautstimulationseinrichtung 5 a nach unten bewegt.

Aus den Querschnitten der Figuren 7 D bis 7 F wird die Bewegung des Stimulationsbereiches durch die koordinierte Ansteuerung der Hautstimulationsbereiche 5 a bis 5 h deutlich. Durch die in den Figuren 7 A bis 7 C gezeigte Abfolge wandert ein Stimulationsbereich, der einen Überdruckbereich 13 sowie zwei an diesen Überdruckbereich 13 oben und unten angrenzende Unterdruckbereiche 16 umfasst, in Richtung des Pfeiles 17 nach unten. Dies ermöglicht eine massagenartige Einwirkung auf die Hautoberfläche, deren Wirkung sich auch in untere Schichten, bis in den Muskelbereich, erstreckt.

Durch einfache Änderungen in der Ansteuerung ist die Bewegung der Stimulationsbereiche 13 und 16, die in den Figuren 7 A bis 7 F nach unten verläuft, umkehrbar. Hierzu muss anstelle der obersten Hautstimulationseinrichtung 5 a lediglich von der untersten Hautstimulationseinrichtung 5 h ausgegangen werden.

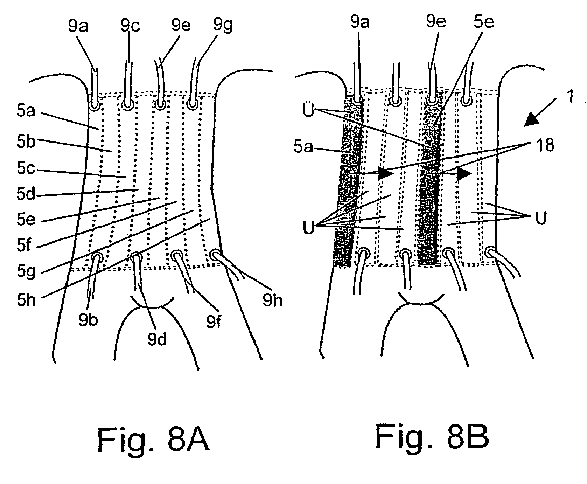

In den Figuren 8 A und 8 B ist ein siebtes Ausführungsbeispiel des erfindungsgemäßen Fitnessgeräts gezeigt, in dem sich im Gegensatz zum Ausführungsbeispiel der Figuren 5, 6 und 7 die Hautstimulationseinrichtungen 5 a - h und damit die diesen Hautstimulationseinrichtungen zugeordneten Stimulationsbereiche sich nicht in Umfangsrichtung, sondern in Längsrichtung des Körpers erstrecken.

Durch gezielte Ansteuerung der Hautstimulationseinrichtungen 5 a - h des Ausführungsbeispiels der Figuren 8 A und 8 D ist eine Bewegung des Stimulationsbereiches in Umfangsrichtung möglich, wenn die einzelnen Hautstimulationseinrichtungen 5 auf ähnliche Weise wie beim Ausführungsbeispiel der Figuren 7 A bis 7 F angesteuert werden.

Dies ist beispielhaft in Figur 8 B dargestellt, wo gezeigt ist, dass sich zwei mit Überdruck beaufschlagte Stimulationsbereiche Ü in Umfangsrichtung des Körpers in Pfeilrichtung 18 bewegen. Bis auf die unterschiedliche Bewegungsrichtung 18 (in Umfangsrichtung anstatt in Richtung 17 nach unten) entspricht der Funktionsablauf der in Figur 8 B gezeigten Betriebsart dem in den Figuren 7 A - 7 F gezeigten Funktionsablauf, so dass hier auf die Beschreibung der Figuren 7 A - 7 F verwiesen werden kann.

Figur 9 zeigt ein achtes Ausführungsbeispiel des erfindungsgemäßen Fitnessgeräts, bei dem die Hautstimulationseinrichtungen 5 in Form von unabhängig ansteuerbaren kleinen Polygonbereichen ausgestaltet sind. Je mehr Hautstimulationseinrichtungen vorgesehen sind und je kleiner diese sind, um so gezielter lassen sich Stimulationsbereiche aus der koordinierten, gleichzeitigen Betätigung mehrerer Hautstimulationseinrichtungen zusammensetzen und über die Körperoberfläche bewegen.

Auch hier ist der Verfahrensablauf genauso, wie beim Ausführungsbeispiel der Figuren 7 A bis 7 F beschrieben. Zur Bewegung eines Stimulationsbereichs um den Körper wird, ausgehend von einer Hautstimulierungseinrichtung 5 oder einer Gruppe von Hautstimulierungseinrichtungen 5, die mit Überdruck beaufschlagt sind, in einer in der beabsichtigten Bewegungsrichtung liegenden Umgebung dieser Hautstimulationseinrichtungen ein Überdruck aufgebaut, während der Druck in den ursprünglich mit Überdruck beaufschlagten Hautstimulationseinrichtungen verringert wird. Dieser Vorgang wird fortgesetzt, so dass sich der Überdruckbereich in eine bestimmte Richtung über den Körper bewegt.

Anstelle der bislang beschriebenen Bewegung eines mit Überdruck beaufschlagten Stimulationsbereichs kann auch ein mit Unterdruck beaufschlagter Stimulationsbereich über den Körper bewegt werden. In diesem Fall sind sämtliche Hautstimulationseinrichtungen 5 eines Fitnessgeräts anfangs mit einem Überdruck beaufschlagt, lediglich eine Hautstimulierungseinrichtung bzw. eine Gruppe davon ist mit Unterdruck beaufschlagt. Ausgehend von diesem Unterdruckbereich werden dann benachbarte Hautstimulierungseinrichtungen, die in der beabsichtigten Bewegungsrichtung des Stimulationsbereichs liegen, mit Unterdruck beaufschlagt.

In Figur 10 ist beispielhaft der fluidische Aufbau eines Fitnessgeräts 1 schematisch dargestellt.

Durch die Pumpvorrichtung 8 wird an einer Saugseite 23 ein Unterdruck und an einer Druckseite 24 ein Überdruck erzeugt.

Über Ventile 25', 25", die von der Steuereinrichtung 26 angesteuert werden, wird jeweils die Saugseite 23 oder die Druckseite 24 mit einer Hautstimulationseinrichtung 5 bzw. 6 verbunden.

Werden die saugseitigen Ventile 25' geöffnet und die druckseitigen Ventile 25" geschlossen, so werden die Hautstimulationseinrichtungen 5 mit Unterdruck beaufschlagt. Beim Schließen der Ventile 25' und Öffnen der Ventile 25" werden die Hautstimulationseinrichtungen 5 mit Überdruck beaufschlagt.

Beim Ausführungsbeispiel der Figur 10 ist jede Hautstimulationseinrichtung 5 mit einer saugseitigen 9' und einer druckseitigen 9" Leitung versehen. Alternativ können anstelle der Einwegventile 25 auch Dreiwegventile (nicht gezeigt) verwendet werden, deren beiden Eingangsanschlüsse mit der Druckseite und der Saugseite und deren Ausgangsseite mit der Hautstimulationseinrichtung verbunden sind. Bei der Verwendung von Dreiwegventilen ist lediglich eine einzige Leitung zu jeder Hautstimulationseinrichtung notwendig, über die dann die Hautstimulationseinrichtung je nach Schaltstellung des Dreiwegventils sowohl mit Überdruck als mit Unterdruck beaufschlagbar ist.

## Patentansprüche

1. Fitnessgerät (1) in Form eines Bekleidungsstückes, mit einer in einem Stimulationsbereich (13, 16) auf die Hautoberfläche eines Trägers des Fitnessgeräts einwirkenden Hautstimulationseinrichtung (5) mit wenigstens einer als Hohlkammer ausgestalteten Druckkammer, durch die in einem Unterdruckzustand im Stimulationsbereich (16) ein auf die Hautoberfläche einwirkender Unterdruck (U) erzeugbar ist und die vom Unterdruckzustand in einen Überdruckzustand, in dem im Stimulationsbereich (13) Überdruck (Ü) auf die Hautoberfläche einwirkt, überführbar ausgestaltet ist, **gekennzeichnet durch** eine Mehrzahl von unabhängig voneinander vom Unterdruckzustand in den Überdruckzustand überführbaren Hautstimulationseinrichtungen und **durch** eine Steuereinrichtung, **durch** welche die Abfolge des Umschaltens einzelner Hautstimulationseinrichtungen (5) vom Unterdruckzustand in den Überdruckzustand koordinierbar ist.

2. Fitnessgerät (1) nach Anspruch 1, **dadurch gekennzeichnet, dass** sich im Überdruckzustand die Hautstimulationseinrichtung (5) an der Hautoberfläche abstützt.

3. Fitnessgerät (1) nach einem der oben genannten Ansprüche, **dadurch gekennzeichnet, dass** im Unterdruckzustand die Hautstimulationseinrichtung (5) von der Hautoberfläche beabstandet ist.

4. Fitnessgerät (1) nach Anspruch **1, dadurch gekennzeichnet, dass** die Hohlkammer zumindest abschnittsweise eine flexible Hülle aufweist.

5. Fitnessgerät (1) nach einem der oben genannten Ansprüche, **dadurch gekennzeichnet, dass** die Hautstimulationseinrichtung (5) ein elastisches Element aufweist, durch das im Überdruckzustand eine Druckkraft auf den Stimulationsbereich ausgeübt ist.

6. Fitnessgerät (1) nach einem der oben genannten Ansprüche, **dadurch gekennzeichnet, dass** wenigstens eine Magnetfeldeinrichtung vorgesehen ist, durch die ein auf den Körper des Trägers des Fitnessgeräts einwirkendes Magnetfeld erzeugbar ist.

7. Fitnessgerät (1) nach einem der oben genannten Ansprüche, **dadurch gekennzeichnet, dass** wenigstens eine Bestrahlungseinrichtung vorgesehen ist, durch die der Körper des Trägers zumindest abschnittsweise mit Licht bestrahlbar ist.

8. Fitnessgerät (1) nach Anspruch 7, **dadurch gekennzeichnet, dass** die Bestrahlungseinrichtung wenigstens einen Lichtleiter aufweist, in dem das Licht von einer Lichtquelle zum Körper des Trägers geleitet ist.

9. Fitnessgerät (1) nach einem der oben genannten Ansprüche, **dadurch gekennzeichnet, dass** eine Klimatisierungseinrichtung vorgesehen ist, durch die die Temperatur des in das Fitnessgerät eingeleiteten Fluids regelbar ist.

10. Fitnessgerät (1) nach einem der oben genannten Ansprüche, **dadurch gekennzeichnet, dass** eine Klimatisierungseinrichtung vorgesehen ist, durch die die Feuchte des in das Fitnessgerät eingeleiteten Fluids regelbar ist.

11. Fitnessgerät (1) nach einem der oben genannten Ansprüche, **dadurch gekennzeichnet, dass** das Fitnessgerät eine flexible, im Stimulationsbereich fluidundurchlässige Hülle aufweist.

## Claims

1. A fitness device (1) in the form of a garment, comprising a skin stimulation means (5) to act on the skin surface of a wearer of the fitness device in a stimulation area (13, 16), said skin stimulation means (5) having at least one pressure chamber configured as a hollow chamber, by which in a negative pressure state in the stimulation area (16) a negative pressure (U) acting on the skin surface can be produced, and which is designed to be transferable from the negative pressure state into an overpressure state in which in the stimulation area (13) overpressure (Ü) acts on the skin surface, **characterized by** a plurality of skin stimulation means that can be transferred independently of one another from the negative pressure state into the overpressure state, and by a controller for coordinating the sequence of the switching of individual skin stimulation means (5) from the negative pressure state into the overpressure state.

2. The fitness device (1) according to claim 1, **characterized in that** the skin stimulation means (5) is supported on the skin surface in the overpressure state.

3. The fitness device (1) according to any one of the aforementioned claims, **characterized in that** in the negative pressure state the skin stimulation means (5) is spaced apart from the skin surface.

4. The fitness device (1) according to claim 1, **characterized in that** the hollow chamber comprises a flexible cover at least in sections.

5. The fitness device (1) according to any one of the aforementioned claims, **characterized in that** the skin stimulation means (5) comprises an elastic element by which in the overpressure state a compressive force is exerted on the stimulation area.

6. The fitness device (1) according to any one of the aforementioned claims, **characterized in that** at least one magnetic field means is provided by which a magnetic field acting on the body of the wearer of the fitness device is producible.

7. The fitness device (1) according to any one of the aforementioned claims, **characterized in that** at least one irradiation means is provided for irradiating the wearer's body with light at least in sections.

8. The fitness device (1) according to claim 7, **characterized in that** the radiation means comprises at least one optical waveguide in which light is guided from a light source to the wearer's body.

9. The fitness device (1) according to any one of the aforementioned claims, **characterized in that** a climate control means is provided, by which the temperature of the fluid passed into the fitness device is controllable.

10. The fitness device (1) according to any one of the aforementioned claims, **characterized in that** a climate control means is provided, by which the moisture of the fluid passed into the fitness device is controllable.

11. The fitness device (1) according to any one of the aforementioned claims, **characterized in that** the fitness device comprises a flexible cover which is fluid-impermeable in the stimulation area.

## Revendications

1. Appareil de fitness (1) en forme de pièce vestimentaire, comprenant un dispositif de stimulation cutanée (5), qui agit dans une région de stimulation (13, 16) sur l'épiderme d'un porteur de l'appareil de fitness, avec au moins un compartiment de pression configuré sous forme de compartiment creux, par lequel peut être produit une dépression (U) agissant sur l'épiderme dans un état de dépression dans la région stimulée (16) et qui a une configuration transférable de l'état de dépression dans un état de surpression, dans lequel une surpression (Ü) agit sur l'épiderme dans la région stimulée (13), **caractérisé par** une pluralité de dispositifs de stimulation cutanée transférables indépendamment les uns des autres de l'état de dépression dans l'état de surpression et par un dispositif de commande, qui permet de coordonner la suite de la commutation de dispositifs de stimulation cutanée individuels (5) de l'état de dépression dans l'état de surpression.

2. Appareil de fitness (1) suivant la revendication 1, **caractérisé en ce que** le dispositif de stimulation cutanée (5) s'appuie sur l'épiderme dans l'état de surpression.

3. Appareil de fitness (1) suivant l'une des revendications précédentes, **caractérisé en ce que** le dispositif de stimulation cutanée (5) est distant de l'épiderme dans l'état de dépression.

4. Appareil de fitness (1) suivant la revendication 1, **caractérisé en ce que** le compartiment creux présente au moins par sections une gaine flexible.

5. Appareil de fitness (1) suivant l'une des revendications précédentes, **caractérisé en ce que** le dispositif de stimulation cutanée (5) comporte un élément élastique, par lequel une force de pression est exercée sur la région stimulée dans l'état de surpression.

6. Appareil de fitness (1) suivant l'une des revendications citées ci-dessus, **caractérisé en ce qu'**il est prévu au moins un dispositif à champ magnétique, qui permet de produire un champ magnétique agissant sur le corps du porteur de l'appareil de fitness.

7. Appareil de fitness (1) suivant l'une des revendications précédentes, **caractérisé en ce qu'**il est prévu au moins un dispositif d'irradiation, qui permet d'irradier de lumière le corps du porteur, au moins par sections.

8. Appareil de fitness (1) suivant la revendication 7, **caractérisé en ce que** le dispositif d'irradiation comporte au moins un guide d'ondes optiques, dans lequel la lumière est guidée d'une source lumineuse en direction du corps du porteur.

9. Appareil de fitness (1) suivant l'une des revendications précédentes, **caractérisé en ce qu'**il est prévu un dispositif de climatisation, qui permet de régler la température du fluide introduit dans l'appareil de fitness.

10. Appareil de fitness (1) suivant l'une des revendications précédentes, **caractérisé en ce qu'**il est prévu un dispositif de climatisation, qui permet de régler l'humidité du fluide introduit dans l'appareil de fitness.

11. Appareil de fitness (1) suivant l'une des revendications précédentes, **caractérisé en ce que** l'appareil de fitness présente une gaine flexible, imperméable au fluide dans la région stimulée.
